# EUROPÄISCHE PATENTSCHRIFT

(11) **EP 3 395 936 B1**
(45) Veröffentlichungstag und Bekanntmachung des Hinweises auf die Patenterteilung: **20.11.2019**
(21) Anmeldenummer: 18169053.8
(22) Anmeldetag: 24.04.2018
(51) Int. Cl.: C12M 1/107, B01F 7/00, B01F 15/00, C12M 1/00, C12M 1/06

(54) **ANLAGE ZUR BEHANDLUNG FERMENTIERBARER SUBSTANZEN**
INSTALLATION FOR TREATING FERMENTABLE SUBSTANCES
INSTALLATION DE TRAITEMENT DE SUBSTANCES FERMENTABLES

(30) Priorität: 28.04.2017 DE 102017109271
(43) Veröffentlichungstag der Anmeldung: 31.10.2018
(73) Patentinhaber: Patent Plus B.V., 5751 PX Deurne (NL)
(72) Erfinder: Roelofs, Seine, 5751 PX Deurne (NL)
(74) Vertreter: Fritz, Edmund Lothar

(56) Entgegenhaltungen:
- EP-A1- 2 463 359
- EP-A1- 2 636 444
- DE-A1-102013 219 938

## Beschreibung

Die vorliegende Erfindung betrifft eine Anlage zur Behandlung fermentierbarer organischer Substanzen umfassend deren vorzugsweise anaerobe Vergärung in wenigstens einem Gärbehälter, wobei oberhalb des Gärbehälters ein Gasdach angeordnet ist, welches das bei der Vergärung gebildete Biogas auffängt, oder der Gärbehälter eine Behälterdecke aufweist, sowie umfassend wenigstens eine Einrichtung, welche das Gärgut im Gärbehälter durchmischt.

Aufgrund der in einiger Zeit zu erwartenden Erschöpfung der natürlichen Erdöl- und Erdgasressourcen ist man auf der Suche nach neuen Energieträgern, die sich dauerhaft nutzen lassen. Interessant sind dabei insbesondere Energieträger, die ein geringeres Gefahrenpotential aufweisen als die Kernkraft und die sich ohne Klimaschädigung nutzen lassen. Eine der bekannten Möglichkeiten, die Nutzung von Wasserstoff, hat den Nachteil, dass es sich um ein an der Luft hochexplosives Gas handelt, dessen Handhabung schwierig ist, so dass diese Technologie ein hohes Gefahrenpotential birgt. Andere bekannte Methoden zur Nutzung alternativer Energieträger in Form der so genannten nachwachsenden Rohstoffe, zum Beispiel die Gewinnung von Rapsölmethylester aus dem Rapsanbau, haben den Nachteil eines vergleichsweise niedrigen Ertrags pro Hektar Anbaufläche und sind daher zum vollständigen Ersatz fossiler Brennstoffe wenig geeignet.

Eine der Methoden zur Gewinnung von Energie aus alternativen Quellen, nämlich die Herstellung von Biogas (im wesentlichen Methan) aus Biomasse, hat in den letzten Jahren weiter an Bedeutung gewonnen. Dabei wird durch anaerobe Vergärung von Biomasse verschiedener Herkunft mittels Mikroorganismen Biogas gewonnen. Als Biomasse kann beispielsweise Gülle eingesetzt werden, die in Agrarbetrieben mit Rinderhaltung in großen Mengen anfällt. Alternativ kann auch beispielsweise Klärschlamm, Mist, Bioabfälle, Speisereste oder Biomasse aus gezielt angebauten Energiepflanzen verarbeitet werden. Es können außerdem andere organische Abfälle jeglicher Art in die Biomasse eingemischt werden. Das Biogas wird dann in der Regel anschließend verstromt und der Strom in das Netz eingespeist. Alternativ wird heute Biogas auch in zunehmendem Umfang nach einer Gasaufbereitung (Methananreicherung) in das öffentliche Gasnetz eingespeist. Die Behandlung des Gärguts erfolgt in meist größeren Behältern, in denen zunächst eine Hydrolyse erfolgt, bei der die Biopolymere enzymatisch in kleinere Bausteine wie Fettsäuren, Zucker, Aminosäuren zerlegt werden, die dann in der nachfolgenden Fermentation von anaeroben Bakterien zu kleinen organischen Säuren und schließlich zu Essigsäure umgesetzt werden. Aus der Essigsäure entsteht dann in der weiteren Umsetzung Methan.

Für die Durchmischung des Gärmediums in Gärbehältern für die Biogasherstellung werden nach dem Stand der Technik heute überwiegend Rührwerke beispielsweise Tauchrührwerke verwendet.

In der DE 10 2007 024 947 A1 ist ein Biogasfermenter beschrieben, bei dem ein axiales Rührwerk zur Umwälzung der Biomasse im Faulraum außerhalb des eigentlichen Fermenters angeordnet ist. Dabei wird Biomasse aus dem Faulraum in Umwälzungsrohre eingesaugt, durch ein in dem Rohr angeordnetes Rührwerk durchmischt und wieder in den Fermenter zurückgepumpt. Es wird hier darauf hingewiesen, dass die Verwendung von Rührwerken, die innerhalb des Faulraums selbst angeordnet sind, nachteilig sein kann. Beispielsweise ist es bei einem Defekt notwendig, den kompletten Faulraum zu entleeren. Außerdem bereitet die Inhomogenität und Zähigkeit der im Faulraum befindlichen Biomasse Probleme bei der Umwälzung mittels herkömmlicher Rührwerke. Die hier beschriebene Lösung schlägt vor, das Rührwerk in den Bereich außerhalb des Faulraums zu verlagern. Da allerdings die Biomasse aus dem Faulraum in Umwälzungsrohre eingesaugt wird und ebenfalls über Rohre wieder in den Faulraum zurückgepumpt wird, ergeben sich strömungstechnische Behinderungen bedingt durch die vergleichsweise geringen Querschnitte dieser Rohre. Außerdem ist bauartbedingt der Anteil der Biomasse, der jeweils eine Durchmischung erfährt, vergleichsweise gering bezogen auf den Gesamtanteil des Volumens der Biomasse im Behälter. Auch neue Biomasse wird bei dieser bekannten Anlage dem Gärbehälter über eine rohrförmige Zuführungsvorrichtung zugeführt, wodurch sich entsprechende Einschränkungen ergeben.

Bei einem Gärbehälter der eingangs beschriebenen Art muss im Falle eines Defekts am Rührwerk das Gasdach bzw. die Behälterdecke des Gärbehälters geöffnet werden, um einen Zugang zu dem Rührwerk zu schaffen. Dies führt zu einem vergleichsweise großen Gasverlust, da das unter dem kuppelförmigen Gasdach angesammelte Biogas verloren geht. Es kommt zudem zu einem längeren Stillstand der Anlage und beim erneuten Anfahren der Anlage muss man darauf achten, dass kein Sauerstoff in das Biogas gelangt, so dass es unter Umständen notwendig ist, die Anlage und die Gasleitung zunächst mit Biogas zu spülen, bevor man Biogas in die Produktgasleitung abführen kann. Das Öffnen des Gasdaches bzw. der Behälterdecke ist somit mit einer erheblichen Betriebsunterbrechung verbunden und auch die damit verbundenen Montagearbeiten sind aufwändig. Das Dokument EP 2 636 444 A1 und auch das Dokument DE 10 2013 219 938 A1 zeigen jeweils eine Anlage nach den Merkmalen des Oberbegriffes des Anspruchs 1.

Hier setzt die vorliegende Erfindung ein. Aufgabe der Erfindung ist es, eine Anlage zur Behandlung fermentierbarer organischer Substanzen der eingangs genannten Gattung zur Verfügung zu stellen, bei der zwar Einrichtungen zur Durchmischung des Gärguts im Gärbehälter selbst angeordnet sind, aber dennoch die Wartung oder Reparatur des Rührwerks möglich ist, ohne das Gasdach insgesamt abzunehmen oder die Behälterdecke zu öffnen.

Die Lösung dieser Aufgabe liefert eine Anlage der eingangs genannten Gattung mit den Merkmalen des Hauptanspruchs.

Erfindungsgemäß wird vorgeschlagen, dass die wenigstens eine Einrichtung zur Durchmischung des Gärguts in einem Bereich des Gärbehälters angeordnet ist, welcher nicht von dem Gasdach oder der Behälterdecke überdeckt ist.

Es gibt im Prinzip zwei Typen von Gärbehältern, wobei bei beiden Typen die vorliegende Erfindung vorteilhaft anwendbar ist. Bei der einen Behältertype erstreckt sich über dem Garbehälter ein kuppelförmiges Gasdach, unter dem das durch die Gärung produzierte Gas aufgefangen wird. Dieses Gasdach kann zum Beispiel aus Kunststoff gefertigt sein. Bei der anderen Behältertype ist kein solches kuppelförmiges Gasdach vorhanden, sondern der Behälter hat eine zumeist flache Behälterdecke, die beispielsweise aus Beton gefertigt sein kann. Das produzierte Biogas wird in diesem Fall meistens direkt aus dem Gärbehälter abgeleitet in einen separaten Gasbehälter. Bei beiden Behältervarianten ergeben sich die gleichen Vorteile, denn das Rührwerk befindet sich in einem Bereich, der außerhalb der Überdeckung durch das Gasdach oder durch die Behälterdecke liegt.

Gemäß der vorliegenden Erfindung ist somit die Einrichtung zur Durchmischung des Gärguts zwar so angeordnet, dass diese das Gärgut im Gärbehälter selbst durchmischt, d.h. diese Einrichtung befindet sich nicht außerhalb des Gärbehälters und auch die Durchmischung findet nicht außerhalb des Gärbehälters statt und wird dann in diesen zurück gefördert, wie dies von anderen Lösungen her bekannt ist. Trotz dieser Durchmischung des Gärguts im Gärbehälter selbst ist jedoch gemäß der Erfindung die Einrichtung zur Durchmischung des Gärguts so angeordnet, dass sie sich nicht unterhalb des Gasdachs oder unterhalb der Behälterdecke des Gärbehälters befindet. Dies hat den wesentlichen Vorteil, dass man einen Zugriff auf diese Einrichtung zur Durchmischung im Falle einer Reparatur oder Wartung hat, ohne dass man dazu das Gasdach oder die Behälterdecke öffnen muss.

Ein weiterer wichtiger Vorteil ergibt sich bei beiden vorgenannten Varianten (mit Gasdach oder Behälterdecke) dadurch, dass man zu Revisionszwecken einen Zugang zu einer Art Kammer außerhalb des Gasdachs oder der Behälterdecke hat, bei dessen Öffnen man nur einen vergleichsweise geringen Teil des in der Anlage produzierten Gases verliert. Diese Kammer ist gegenüber dem übrigen Gärraum abgetrennt, so dass das dort produzierte Gas im Behälter erhalten bleibt und es auch nicht notwendig ist, die Anlage vor dem wieder verschließen mit Gas zu spülen. Diese Kammer hat ein viel geringeres Volumen im Vergleich zu dem Volumen des Gasraums des gesamten Garbehälters. Dies ist ein ganz wesentlicher Vorteil, denn bei herkömmlichen Anlagen bedeutete ein Öffnen des Gasdachs oder der Behälterdecke immer den Verlust des gesamten Gases, welches sich in dem Gasraum über dem Garmedium befand.

Mit anderen Worten, das ansonsten den Gärbehälter überdeckende Gasdach bzw. die Behälterdecke spart quasi den Bereich, in dem sich die Einrichtung zur Durchmischung befindet aus. Dabei ist es natürlich vorteilhaft, wenn sich die Einrichtung zur Durchmischung in einem peripheren Bereich oder Randbereich des Gärbehälters befindet, so dass man vom Rand des Gärbehälters her diese Einrichtung gut erreichen kann.

Insbesondere ist gemäß einer bevorzugten Ausführungsvariante der Erfindung die Einrichtung zur Durchmischung des Gärguts in der Nähe der äußeren Wandung des Gärbehälters angeordnet. Bei dieser Variante ist es einfacher, das Gasdach bzw. die Behälterdecke so zu formen, dass es diesen Randbereich des Gärbehälters ausspart, d.h. dass das Gasdach bzw. die Behälterdecke quasi um den Bereich herum läuft, in dem sich die Einrichtung zur Durchmischung des Gärguts befindet. Andererseits kann man bei einer Reparatur von außen her die Einrichtung zur Durchmischung gut erreichen, da sie sich nicht weit von der Außenwand entfernt befindet.

Besonders bevorzugt ist die Einrichtung zur Durchmischung des Gärguts in ihrem oberen Bereich von einer Kammer aufgenommen, die jeweils zu den Seiten hin über Trennwände zum Innenraum des Gärbehälters hin abgetrennt ist, wobei die Kammer jedoch unterseitig offen ausgebildet ist. Eine solche Kammer schafft einen separaten Raum, der von dem Gasraum über dem Gärgut im Gärbehälter zu den Seiten hin abgetrennt ist. Diese Kammer befindet sich außerhalb des Umrisses des Gasdachs oder der Behälterdecke, aber dennoch noch innerhalb des Umriss des Gärbehälters, welcher durch dessen Außenwand vorgegeben wird. Das im Gärbehälter erzeugte Biogas sammelt sich unter dem Gasdach oder wird bei einem Gärbehälter mit flacher Behälterdecke abgeleitet. Bei der ersten Variante weicht somit die Grundrissform des unteren Bereichs des Gasdachs von der Grundrissform des von dem Gasdach überdeckten Gärbehälters ab, da von dem Grundriss des Gasdachs der Bereich ausgespart ist, in dem sich die genannte Kammer befindet. Beispielsweise könnte der Gasbehälter im Prinzip zylindrisch sind, so dass er einen etwa kreisförmigen Grundriss hat, wohingegen das Gasdach so geformt ist, dass es an mindestens einer Stelle, nämlich dort wo sich die Kammer befindet, eine Abweichung vom kreisförmigen Grundriss hat, durch eine konkave Aussparung. Gleiche Überlegungen gelten für die flache Behälterdecke.

Die vorgenannten Trennwände der Kammer können auch beispielsweise beweglich ausgebildet sein, d.h. bei einer Wartung könnte man zum Beispiel eine solche Trennwand nach oben ziehen und später wieder hinab lassen.

Wenn der Gärbehälter beispielsweise im Grundriss etwa rechteckig ist, könnte der Grundriss des Gasdachs oder der Behälterdecke beispielsweise eine runde, rechteckige, dreieckige, polygonale oder auch beliebig anders geformte Aussparung mit kurvenförmiger oder eckiger Begrenzungslinie aufweisen.

Wenn man sich Zugang zu der genannten Kammer verschafft, bleibt dabei das Gasdach bzw. die Behäterdecke geschlossen, es reicht aus, wenn man sich von oben her, beispielsweise über eine Öffnung, Zugang zu der Kammer verschafft. Unterseitig ist die Kammer offen, was bedeutet, dass hier eine Verbindung zu dem Gasraum über dem Gärgut besteht. Etwas Gas gelangt somit in die Kammer und wenn man diese im Falle einer Reparatur öffnet, geht dieses Gas verloren. Jedoch ist das Volumen der Kammer sehr klein im Verhältnis zum Gesamtvolumen unter dem Gasdach des Gärbehälters. Zum einen kann die Kammer im Grundriss viel kleiner sein als der Gärbehälter, denn im Prinzip genügt es, wenn die Kammer Teile des Rührwerks aufnimmt und eine ausreichende Größe für Wartungs- oder Reparaturarbeiten an dem Rührwerk aufweist. Außerdem kann die Kammer quasi als Revisionsöffnung für eine Inspektion des Behälterinnenraums dienen. Zweitens ist es auch ausreichend, wenn die Höhe der Kammer nur einen Bruchteil der gesamten Höhe des Gärbehälters ausmacht, denn die Kammer erstreckt sich in Höhenrichtung nur im oberen Bereich des Gasraums.

Vorzugsweise ist die Kammer zudem im oberen Endbereich der Außenwand des Behälters angeordnet, also ungefähr in der Höhe, in der die Außenwand endet und das Gasdach beginnt. In diesem Bereich ist es am einfachsten, sich Zugang zum Rührwerk zu verschaffen. Da die Kammer bevorzugt an ihrer Unterseite offen ist, kann als Einrichtung zur Durchmischung des Gärguts ein Rührwerk verwendet werden, welches unterseitig aus der Kammer nach unten hin herausragt und in das Gärgut eintaucht. Gemeint ist an dieser Stelle, dass die Kammer bevorzugt so angeordnet ist, dass sie oberseitig zumindest etwa mit dem oberen Ende der Außenwand des Gärbehälters endet, so dass dort ein bequemer Zugang zu der Kammer besteht. Es ist alternativ aber auch möglich, die Kammer tiefer, d.h. unterhalb des oberen Endes der Außenwand anzuordnen. In diesem Fall müsste man aber im Bereich der Kammer eine Öffnung durch die Außenwand des Gärbehälters vorsehen, was einen höheren Aufwand bedingt. Zudem muss dann sichergestellt sein, dass der Zugang zu der Kammer in jedem Fall oberhalb des höchsten möglichen Pegels des Gärguts im Gärbehälter liegt.

Erfindungsgemäß können zumindest Teile einer Antriebsvorrichtung für die Betätigung der Einrichtung zur Durchmischung des Gärguts innerhalb der Kammer verlaufen und/oder aus der Kammer nach unten hinausragen oder zumindest eine Halterung, an der sich wenigstens ein Rührwerk befindet, kann durch die Kammer nach unten hin verlaufen und bevorzugt auch über die obere Öffnung der Kammer hinaus nach oben ragen, so dass man über diese Halterung einen Zugriff für die Handhabung des Rührwerks hat.

Vorzugsweise weist die Kammer eine obere Abdeckung auf, welche zum Öffnen der Kammer von oben her abnehmbar oder aufklappbar oder schwenkbar ausgebildet ist. Im Betriebszustand ist die Kammer damit zur Atmosphäre hin geschlossen, so dass hier kein Biogas entweichen kann. Wird die Klappe beispielsweise durch Anheben oder Schwenken (bei gelenkiger Aufhängung der Klappe) geöffnet, entsteht eine ausreichend große Öffnung zum Innenraum des Gärbehälters, die unter anderem einen Zugriff auf das Rührwerk ermöglicht.

Weiterhin weist vorzugsweise der Gärbehälter im Bereich der Kammer eine etwa horizontale begehbare Plattform auf, über die ein Zugang zu der Kammer gegeben ist. Personen, die mit einer Reparatur des Rührwerks befasst sind, können sich dann auf dieser Plattform aufhalten, nach Öffnen der Abdeckung das Rührwerk inspizieren und die Arbeiten am Rührwerk vornehmen oder dieses gegebenenfalls aus dem Gärbehälter herausheben. Geräte, Werkzeuge und Arbeitsmaterialien können auf der Plattform abgestellt werden. Das Gasdach bzw. die Behälterdecke beginnen erst jenseits der Kammer, es läuft quasi um die Kammer herum und spart diese aus, so dass das Gasdach bzw. die Behälterdecke für die Arbeiten am Rührwerk nicht geöffnet werden und kein Gas verloren geht.

Entsprechend der vorliegenden Erfindung weist die Grundrisslinie des Gasdachs bzw. der Behälterdecke im Bereich der Kammer eine konkave Einbuchtung auf. Durch das Aussparen des Gasdachs im Bereich der Kammer ergibt sich eine komplexere dreidimensionale Form des Gasdachs, verglichen mit einem herkömmlichen kuppelförmigen Gasdach. Durch geeignete spezielle kunststofftechnische Verfahren ist es aber ohne weiteres möglich, diese Form des Gasdachs zu erzeugen. In der Regel besteht ein Kunststoff-Gasdach aus einem thermoplastischen Polymeren wie beispielsweise PVC oder dergleichen. Durch Formgebungsverfahren kann ein solches Kunststoff-Gasdach hergestellt werden, entweder in einem Stück oder aus mehreren kleineren Teilen, die dann anschließend durch Verschweißen, Vernähen oder andere geeignete Techniken miteinander verbunden werden können.

Grundsätzlich sind dem Fachmann geeignete Methoden zur Herstellung derart komplexer Bauteile aus Kunststoff, wie es das erfindungsgemäß geformte Gasdach darstellt, bekannt. Im Rahmen der vorliegenden Erfindung wurde ein Prototyp aus mehreren über eine Computersteuerung exakt passend zugeschnittenen Einzelteilen hergestellt, die dann zusammengefügt und miteinander verschweißt und/oder vernäht wurden. Grundsätzlich ist auch beispielsweise die Herstellung des Gasdachs in mehreren Segmenten im 3D-Druck möglich. Alternativ käme eine Herstellung mehrerer Segmente durch Tiefziehen in Betracht. Ebenso möglich ist die Herstellung aus faserverstärkten Kunststoffen über Gewebe, Gelege oder Rovings, wobei auch hier einzelne Teilsegmente des Gasdachs hergestellt und dann miteinander verbunden werden können.

Die in den Unteransprüchen genannten Merkmale betreffen bevorzugte Weiterbildungen der erfindungsgemäßen Aufgabenlösung. Weitere Vorteile der Erfindung ergeben sich aus der nachfolgenden Detailbeschreibung.

Nachfolgend wird die vorliegende Erfindung anhand von Ausführungsbeispielen unter Bezugnahme auf die beiliegenden Zeichnungen näher erläutert. Dabei zeigt:

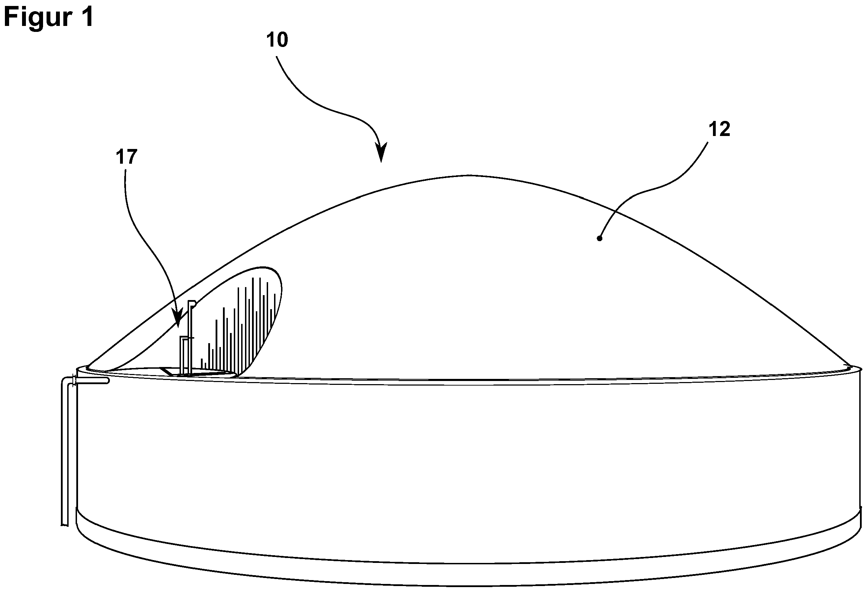

Figur 1 eine Ansicht eines erfindungsgemäßen Gärbehälters mit montiertem Gasdach;
Figur 2 eine perspektivische Teilansicht das Gärbehälters im Bereich der Kammer bei abgenommenem Gasdach;
Figur 3 eine vergrößerte perspektivische Detailansicht der Kammer;
Figur 4 einen Teilausschnitt einer schematischen Draufsicht auf den Gärbehälter bei abgenommenem Gasdach.

Es wird zunächst auf die Figur Bezug genommen und anhand dieser wird der grundsätzliche Aufbau eines erfindungsgemäßen Gärbehälters 10 erläutert. Dieser Gärbehälter 10 umfasst eine beispielsweise im Umriss rundes und somit in der Grundform etwa zylindrisches Becken 11, welches das Gärgut aufnimmt, sowie ein über dem Becken 11 angeordnetes Gasdach 12, welches die Form einer Art Kuppel aufweist und das Becken 11 oberseitig weitgehend vollständig überdeckt, mit Ausnahme eines ausgesparten Bereichs, in dem sich die Kammer befindet, der später noch näher erläutert wird. Dadurch kann das durch Fermentation des Gärguts im Gärbehälter entstehende und nach oben steigende Biogas unterhalb des Gasdachs aufgefangen und in an sich bekannter Weise gewonnen und beispielsweise über Gasleitungen abgeleitet werden. Jedoch hat das Gasdach 12 in dem Bereich, in dem sich die Kammer befindet eine konkave Einbuchtung 13, so dass der Bereich der Kammer quasi von dem Gasdach ausgespart ist und auch bei montiertem Gasdach ein Zugang zu der Kammer gegeben ist, ohne dass es notwendig ist, das Gasdach von dem Gärbehälter abzunehmen.

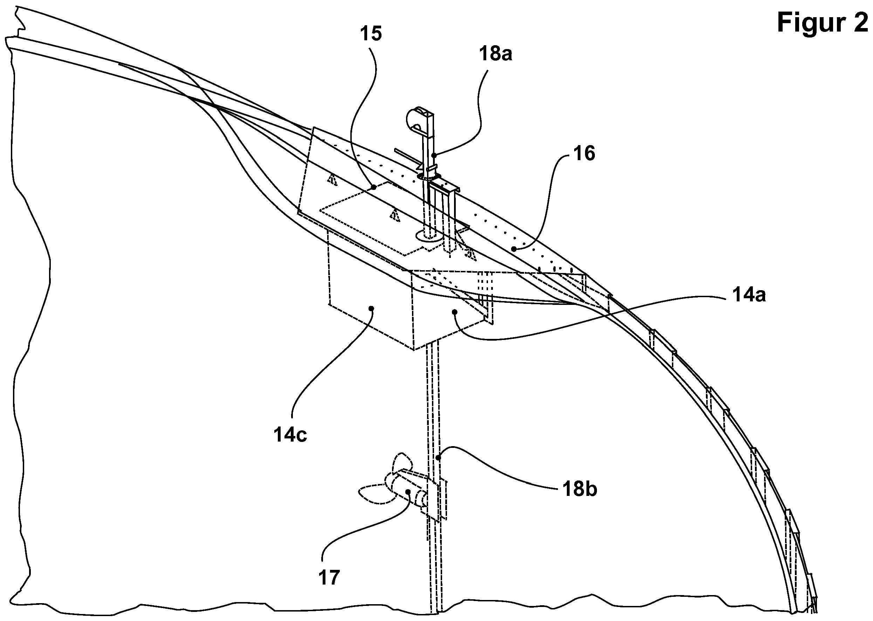

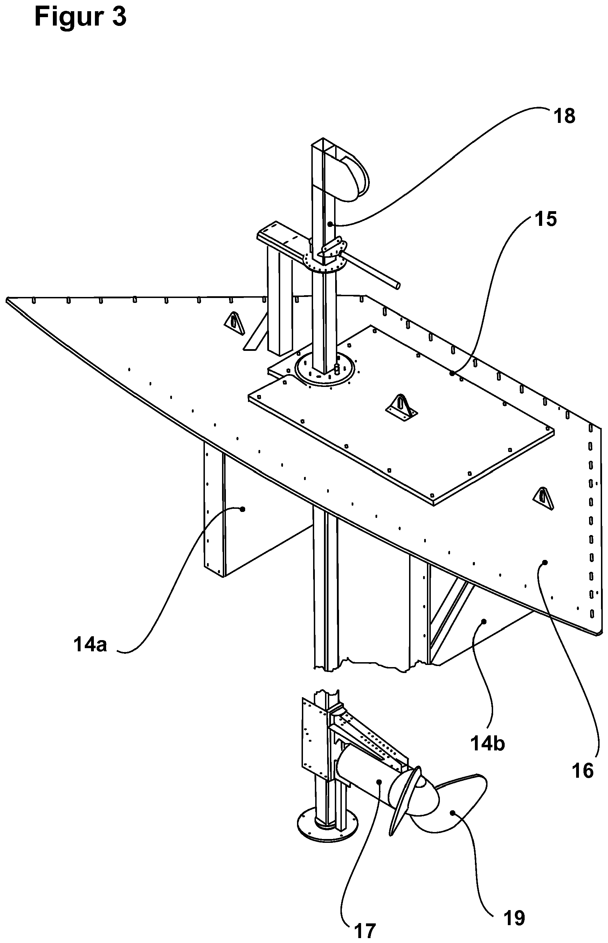

Nachfolgend wird nun die vorliegende Erfindung unter Bezugnahme auf die Figuren 2 und 3 näher erläutert. Die perspektivische Ansicht gemäß Figur 2 zeigt einen Teilausschnitt im Randbereich des Beckens 11 des Gärbehälters bei abgenommenem Gasdach. Man erkennt, dass sich am Umfang des Beckens 11 innenseitig im oberen Bereich die Kammer 14 befindet, die beispielsweise einen etwa rechteckigen (oder auch anders geformten) Umriss aufweist, mit zwei Seitenwänden 14 a, 14 b, die etwa radial in das Becken hineinragen und einer weiteren Seitenwand 14 c, die quer zu den beiden anderen Seitenwänden 14 a, 14 b und im Prinzip in Richtung einer Sekante zu dem im Grundriss etwa kreisförmigen Becken 11 verläuft. Oberseitig weist die Kammer 14 eine Abdeckung 15 auf, die sich öffnen lässt, so dass man von oben her einen Zugang zum Inneren der Kammer hat, wenn man beispielsweise eine Wartung oder Reparatur am Rührwerk vornehmen will. In Höhe der Abdeckung 15 ist außerdem eine horizontale Plattform 16 vorgesehen, die begehbar ist, so dass Personen über diese Plattform Zugang zu der Kammer 14 haben und die Abdeckung 15 abnehmen können, um so Zugang zum Inneren der Kammer 14 und zu dem Rührwerk 17 zu erhalten.

Das Rührwerk 17 befindet sich an einer Halterung 18, die etwa vertikal vom Rührwerk 17 aus nach oben hin verläuft. Diese Halterung 18 hat einen obersten Teilabschnitt 18 a, der sich oberhalb der Plattform 16 erstreckt und einen sich dann nach unten hin anschließenden Abschnitt, der in Figur 2 nicht erkennbar ist, da sich die Halterung 18 zunächst durch die Kammer 14 hindurch erstreckt und einen unteren Abschnitt 18 b, der bevorzugt etwa vertikal in das Becken 11 hineinragt und an dem das Rührwerk 17 befestigt ist, in einer Höhe, die in das Gärgut im Becken eintaucht. Teile des Antriebsvorrichtung oder der Steuerung für das Rührwerks 17 (wie zum Beispiel Verbindungsleitungen) können so an der Halterung 18 angebracht sein und beispielsweise durch die Halterung nach oben hin verlaufen. Das Rührwerk 17 ist deshalb an der Halterung 18 angebracht, damit man im Falle eines Defekts die Abdeckung 15 öffnen und beispielsweise die Halterung 18 mit dem Rührwerk nach oben hin ziehen und so das Rührwerk 17 aus dem Gärbehälter herausnehmen und Reparaturen an diesem vornehmen kann, ohne dass man dazu das Gasdach öffnen muss, denn die Abdeckung 15 der Kammer 14 und die Plattform 16 befinden sich außerhalb des Gasdachs.

In der vergrößerten Darstellung gemäß Figur 3 erkennt man, dass die die Abdeckung 15 der Öffnung der Kammer umgebende Plattform 16 an ihrem äußeren Rand etwa entlang der Umrissform des runden Behälters und somit etwas abgerundet verläuft und dort etwas breiter ist als an ihrem inneren Rand. Man erkennt die Halterung 18, die mit ihrem oberen Abschnitt über die Plattform 16 nach oben hin ragt, so dass man nach Öffnen der Abdeckung 15 mit einer geeigneten Vorrichtung wie beispielsweise einem Kran die Halterung 18 und damit das Rührwerk 17 nach oben aus der Kammer 14 heraus ziehen kann. Man sieht in Figur 3 auch zwei der Seitenwände 14 a, 14 b der Kammer, wobei die Querwand 14 c hier nicht erkennbar ist. Man kann jedoch gut erkennen, dass die Kammer 14 keinen Boden hat, sondern nach unten hin offen ist, so dass die Halterung 18 mit dem Rührwerk 17 nach unten aus der Kammer 14 hinausragen und das Rührwerk 17 in das Gärgut hineinragen kann. Das Rührwerk 17 ist in dem Ausführungsbeispiel ein Tauchrührwerk, dessen Achse etwa horizontal ausgerichtet ist und das wie eine Art Propeller 19 mit Flügeln am vorderen Ende aufweist, der motorisch angetrieben wird. Somit ist die Achse des Rührwerks 17 etwa rechtwinklig zur der etwa senkrecht verlaufenden Halterung 18 ausgerichtet, an der das Rührwerk angebracht ist.

Wenn somit der Betreiber der Anlage eine Wartung oder Reparatur im Bereich des Rührwerks 17 vornehmen möchte, dann genügt es, wenn die obere Abdeckung 15 der Kammer 14 angehoben wird, so dass dann eine Öffnung gegeben ist, die den Zugang zum Inneren der Kammer 14 und somit auch zum Rührwerk und zu dem unter der nach unten hin offenen Kammer 14 liegenden Innenraum des Gärbehälters 10 schafft. Da das Volumen der Kammer 14 wie man aus Figur 2 erkennt viel kleiner ist als das Gesamtvolumen des Behälters, geht beim Öffnen der Kammer lediglich ein vergleichsweise kleiner Anteil des im Gärbehälter erzeugten Biogases verloren. Es ist somit nicht nötig, das Gasdach 12 zu öffnen, wodurch ein erheblicher größerer Anteil des Biogases verloren ginge. Das Gasdach 12 bleibt vielmehr beim Öffnen der Kammer 14 geschlossen.

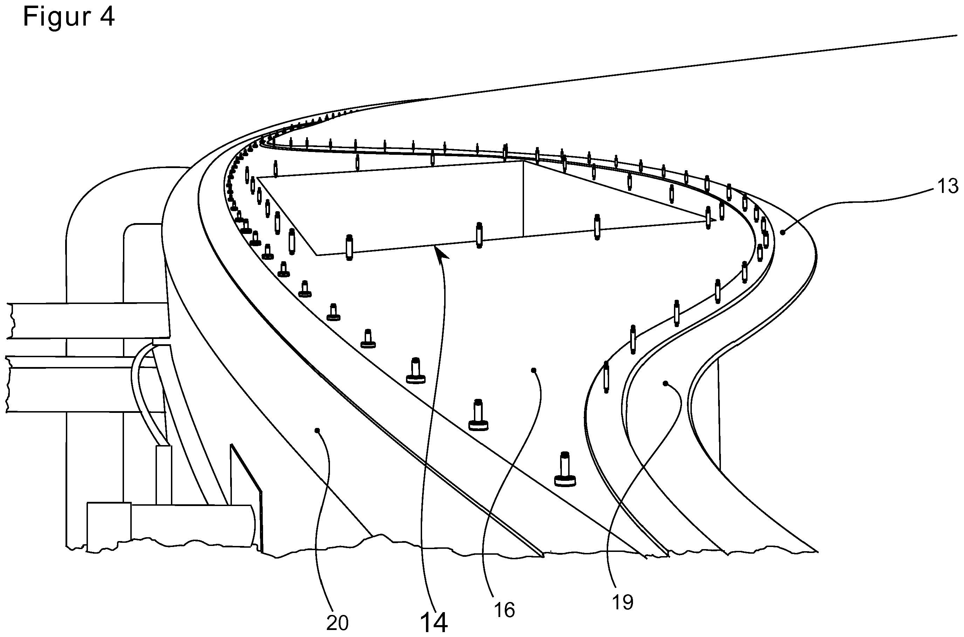

Die Form des Gasdaches und die Position der Kammer 14 sind aus Figur 4 erkennbar. Man sieht, dass dort in der Draufsicht gesehen die Kammer 14 zwischen einer Außenwand 20 und einem inneren Auflagerahmen für das Gasdach des Gärbehälters angeordnet ist, wobei dieser innere Auflagerahmen 19 im Bereich der Kammer 14 eine konkave Einbuchtung aufweist und somit um die Kammer 14 herumläuft. Die untere Umrissform des Gasdachs, das in Figur 4 abgenommen ist und somit nicht eingezeichnet ist, entspricht der Form des inneren Auflagerahmens 19 und hat hier auch die konkave Einbuchtung, so dass der Bereich der Kammer 14 außerhalb des Gasdachs liegt.

Wenn man das Gasdach in der Draufsicht sieht, liegt dieses über den größten Teil seines Umfangs etwa im Bereich der Außenwand 20 des Gärbehälters auf und folgt dessen Umrissform, so dass sich weitgehend ein kreisförmiger Umriss und eine konvexe Kuppelform für das Gasdach ergeben und sich das erzeugte Biogas unter der Kuppel sammeln kann. Nur in dem Bereich, in dem sich die Kammer 14 befindet, ist der untere Randbereich des Gasdachs konkav nach innen hin eingebuchtet, so dass sich das Volumen unterhalb des Gasdachs etwas verringert und die Kammer nicht von dem Gasdach überdeckt wird, sondern außerhalb liegt.

### BEZUGSZEICHENLISTE

- 10: Gärbehälter
- 11: Becken
- 12: Gasdach
- 13: Einbuchtung
- 14: Kammer
- 14 a: Seitenwand
- 14 b: Seitenwand
- 14 c: Seitenwand in Querrichtung
- 15: Abdeckung
- 16: Plattform
- 17: Rührwerk
- 18: Halterung
- 19: innerer Auflagerahmen
- 20: Außenwand

## Patentansprüche

1. Anlage zur Behandlung fermentierbarer organischer Substanzen umfassend deren vorzugsweise anaerobe Vergütung in wenigstens einem Gärbehälter (10), wobei oberhalb des Gärbehälters ein Gasdach (12) angeordnet ist, welches das bei der Vergärung gebildete Biogas auffängt, oder der Gärbehälter (10) eine Behälterdecke aufweist, sowie umfassend wenigstens eine Einrichtung (17), welche das Gärgut im Gärbehälter durchmischt, wobei die wenigstens eine Einrichtung (17) zur Durchmischung des Gärguts in einem Bereich des Gärbehälters (10) angeordnet ist, welcher nicht von dem Gasdach (12) oder der Behälterdecke überdeckt ist, wobei die Einrichtung (17) zur Durchmischung des Gärguts in ihrem oberen Bereich von einer Kammer (14) aufgenommen ist, die jeweils zu den Seiten hin über Trennwände (14 a, 14 b, 14 c) zum Innenraum des Gärbehälters hin abgetrennt ist, wobei die Kammer (14) jedoch unterseitig offen ausgebildet ist, wobei das Gasdach (12) oder die Behälterdecke eine Umrissform aufweisen, die den Bereich, in dem sich die Kammer (14) befindet, ausschließt, wobei die Grundrisslinie des auf den Gärbehälter (10) aufsetzbaren Gasdachs (12) bzw. der Behälterdecke um die Kammer (14) herumläuft, **dadurch gekennzeichnet, dass** die Grundrisslinie des Gasdachs (12) bzw. der Behälterdecke im Bereich der Kammer (14) eine konkave Einbuchtung (13) aufweist.

2. Anlage nach Anspruch 1, **dadurch gekennzeichnet, dass** die Einrichtung (17) zur Durchmischung des Gärguts in einem peripheren Bereich oder Randbereich des Gärbehälters (10) angeordnet ist.

3. Anlage nach Anspruch 1 oder 2, **dadurch gekennzeichnet, dass** die Einrichtung (17) zur Durchmischung des Gärguts in der Nähe der äußeren Wandung (11) des Gärbehälters (10) angeordnet ist.

4. Anlage nach Anspruch 1, **dadurch gekennzeichnet, dass** die Einrichtung zur Durchmischung des Gärguts ein Rührwerk (17) umfasst, welches unterseitig aus der Kammer (14) nach unten hin herausragt.

5. Anlage nach einem der Ansprüche 1 oder 4, **dadurch gekennzeichnet, dass** die Kammer (14) eine obere Abdeckung (15) aufweist welche zum Öffnen der Kammer (14) von oben her abnehmbar oder aufklappbar oder schwenkbar ausgebildet ist.

6. Anlage nach einem der Ansprüche 1 bis 5, **dadurch gekennzeichnet, dass** der Gärbehälter (10) im Bereich der Kammer (14) eine etwa horizontale begehbare Plattform (16) aufweist, über die ein Zugang zu der Kammer (14) gegeben ist.

7. Anlage nach Anspruch 6, **dadurch gekennzeichnet, dass** die Plattform (16) die obere Abdeckung (15) mit der Öffnung zu der Kammer (14) mindestens teilweise umgibt.

8. Anlage nach einem der Ansprüche 1 bis 7, **dadurch gekennzeichnet, dass** zumindest Teile einer Antriebsvorrichtung für die Betätigung der Einrichtung zur Durchmischung des Gärguts innerhalb der Kammer (14) verlaufen und/oder aus der Kammer (14) nach unten hinausragen.

9. Anlage nach einem der Ansprüche 4 bis 8, **dadurch gekennzeichnet, dass** das Rührwerk (17) ein Tauchrührwerk umfasst, welches in das im Gärbehälter befindliche Gärgut eintaucht und welches an einer Halterung (18) angebracht ist, die sich teilweise in der Kammer (14) erstreckt.

10. Anlage nach Anspruch 9, **dadurch gekennzeichnet, dass** ein oberer Abschnitt der Halterung (18) über die Abdeckung (15) und/oder die begehbare obere Plattform der Kammer (14) nach oben hin hinausragt.

11. Anlage nach einem der Ansprüche 1 bis 10, **dadurch gekennzeichnet, dass** das Gasdach aus Kunststoff in mehreren zunächst separaten Segmenten gefertigt ist, welche miteinander verbunden sind, insbesondere verschweißt und/oder vernäht sind.

## Claims

1. An installation for the treatment of fermentable organic substances, comprising the preferably anaerobic fermentation thereof in at least one fermentation vessel (10), wherein a gas cover (12) is disposed above the fermentation vessel (10) which traps the biogas formed during fermentation, or the fermentation vessel (10) comprises a vessel cover, and also comprising at least one device (17) which thoroughly mixes the fermentation material in the fermentation vessel, wherein the at least one device (17) for thoroughly mixing the fermentation material is disposed in a region of the fermentation vessel (10) which is not covered over by the gas cover (12) or the vessel cover, wherein the upper region of the device (17) for thoroughly mixing the fermentation material is accommodated in a chamber (14) which is separated from the interior of the fermentation vessel via separating walls (14a, 14b, 14c) on the respective sides, wherein, however, the underside of the chamber (14) is configured in a manner such that it is open, wherein the shape of the outline of the gas cover (12) or the vessel cover does not include the region in which the chamber (14) is located, whereupon the ground plan outline of the gas cover (12) or the vessel cover which can be placed on the fermentation vessel (10) leads around the chamber (14), **characterized in that** the ground plan outline of the gas cover (12) or of the vessel cover has a concave indentation (13) in the region of the chamber (14).

2. The installation as claimed in claim 1, **characterized in that** the device (17) for thoroughly mixing the fermentation material is disposed in a peripheral region or edge region of the fermentation vessel (10).

3. The installation as claimed in claim 1 or claim 2, **characterized in that** the device (17) for thoroughly mixing the fermentation material is disposed in the vicinity of the outer wall (11) of the fermentation vessel (10).

4. The installation as claimed in claim 1, **characterized in that** the device for thoroughly mixing the fermentation material comprises a stirring device (17) which protrudes downwardly out of the underside of the chamber (14).

5. The installation as claimed in claim 1 or claim 4, **characterized in that** the chamber (14) comprises an upper covering (15) which is configured so as to be upwardly removable or hingeable or pivotable in order to open the chamber (14).

6. The installation as claimed in one of claims 1 to 5, **characterized in that** the fermentation vessel (10) comprises an approximately horizontal walkway platform (16) in the region of the chamber (14) which provides access to the chamber (14).

7. The installation as claimed in claim 6, **characterized in that** the platform (16) at least partially surrounds the upper covering (15) with the opening to the chamber (14).

8. The installation as claimed in one of claims 1 to 7, **characterized in that** at least portions of a drive means for actuating the device for thoroughly mixing the fermentation material run inside the chamber (14) and/or protrude downwardly out of the chamber (14).

9. The installation as claimed in one of claims 4 to 8, **characterized in that** the stirring device (17) comprises a submersible stirring device which is immersed in the fermentation material present in the fermentation vessel and which is attached to a fixture (18), part of which extends in the chamber (14).

10. The installation as claimed in claim 9, **characterized in that** an upper section of the fixture (18) protrudes upwardly over the covering (15) and/or the walkway upper platform of the chamber (14).

11. The installation as claimed in one of claims 1 to 10, **characterized in that** the gas cover is produced from synthetic material in a plurality of initially separate segments which are connected together, in particular welded and/or seamed.

## Revendications

1. Installation de traitement de substances organiques fermentables, dans laquelle ces dernières subissent une valorisation, préférentiellement de nature anaérobie, dans une cuve de fermentation (10), une toiture étanche aux gaz (12) étant disposée au-dessus de la cuve de fermentation et servant à retenir le biogaz généré lors de la fermentation, la cuve de fermentation (10) pouvant également être pourvue d'un plafond de cuve, et laquelle comprend au moins un dispositif (17) destiné à mélanger les matières à fermenter au sein de la cuve de fermentation, l'au moins un dispositif (17) destiné à mélanger les matières à fermenter étant disposé dans une zone de la cuve de fermentation (10) qui n'est pas recouverte par la toiture étanche aux gaz (12) ou par le plafond de cuve, la partie supérieure du dispositif (17) destiné à mélanger les matières à fermenter étant logée dans une chambre (14) dont chacun des côtés est séparé de l'intérieur de la cuve de fermentation par des cloisons (14a, 14b, 14c) alors que la chambre (14) est réalisée de manière à être ouverte vers le bas, la toiture étanche aux gaz (12) ou le plafond de cuve présentant un contour dont la forme n'englobe pas la zone dans la laquelle la chambre (14) est située, la ligne de plan de niveau de la toiture étanche aux gaz (12) ou du plafond de cuve destiné(e) à être placé(é) sur la cuve de fermentation (10) contournant la chambre (14), **caractérisée en ce que** la ligne de plan de niveau de la toiture étanche aux gaz (12) ou du plafond de cuve présente au niveau de la chambre (14) une échancrure (13) concave.

2. Installation selon la revendication 1, **caractérisée en ce que** le dispositif (17) destiné à mélanger les matières à fermenter est disposé dans une zone périphérique ou marginale de la cuve de fermentation (10) .

3. Installation selon les revendications 1 ou 2, **caractérisée en ce que** le dispositif (17) destiné à mélanger les matières à fermenter est disposé à proximité de la paroi extérieure (11) de la cuve de fermentation (10).

4. Installation selon la revendication 1, **caractérisée en ce que** le dispositif destiné à mélanger les matières à fermenter comprend un organe d'agitation (17) lequel dépasse la face inférieure de la chambre (14) de manière à en faire saillie vers la bas.

5. Installation selon l'une des revendications 1 à 4, **caractérisée en ce que** la chambre (14) présente une couverture (15) supérieure laquelle est réalisée de manière à pouvoir l'enlever depuis le haut ou l'ouvrir par un basculement ou pivotement, pour ainsi ouvrir la chambre (14).

6. Installation selon l'une des revendications 1 à 5, **caractérisée en ce que** la cuve de fermentation (10) présente au niveau de la chambre (14) une plateforme horizontale (16), dimensionnée pour marcher dessus, laquelle permet d'accéder à la chambre (14).

7. Installation selon la revendication 6, **caractérisée en ce que** la plateforme (16) entoure, au moins sur certaines parties, la couverture supérieure (15) pourvue d'une ouverture vers la chambre (14)

8. Installation selon l'une des revendications 1 à 7, **caractérisée en ce qu'**un organe moteur permettant d'actionner le dispositif destiné à mélanger les matières à fermenter, s'étend au moins partiellement à l'intérieur de la chambre (14) et/ou fait saillie de la chambre (14) vers le bas.

9. Installation selon l'une des revendications 4 à 8, **caractérisée en ce que** l'organe d'agitation (17) comprend un organe d'agitation plongeur qui est immergé dans les matières à fermenter se trouvant dans la cuve de fermentation et qui est fixé à un support (18) s'étendant partiellement dans la chambre (14).

10. Installation selon la revendication 9, **caractérisée en ce qu'**une partie supérieure du support (18) dépasse la couverture (15) et/ou la plateforme supérieure, dimensionnée pour marcher dessus, de la chambre (14) en en faisant saillie vers le haut.

11. Installation selon l'une des revendications 1 à 10, **caractérisée en ce que** la toiture étanche aux gaz est fabriquée en matière plastique à partir de plusieurs segments, d'abord séparés, lesquels sont joints les uns aux autres, notamment par soudure et/ou couture.
